# EUROPÄISCHE PATENTSCHRIFT

(11) **EP 1 150 738 B1**
(45) Veröffentlichungstag und Bekanntmachung des Hinweises auf die Patenterteilung: **13.11.2002**
(21) Anmeldenummer: 00909195.0
(22) Anmeldetag: 17.02.2000
(51) Int. Cl.: A61M 31/00

(54) **IMPLANTAT**
IMPLANT
IMPLANT

(30) Priorität: 18.02.1999 DE 19907006; 09.03.1999 DE 19910188; 08.10.1999 DE 19948783
(43) Veröffentlichungstag der Anmeldung: 07.11.2001
(73) Patentinhaber: AlCove Surfaces GmbH, 45966 Gladbeck (DE)
(72) Erfinder: BRANDAU, Wolfgang, D-48161 Münster (DE); FISCHER, Alfons, D-45239 Essen (DE); SAWITOWSKI, Thomas, D-45133 Essen (DE); SCHMID, Günter, D-42555 Velbert (DE)
(74) Vertreter: Gesthuysen, von Rohr & Eggert
(86) Internationale Anmeldenummer: EP0001287
(87) Internationale Veröffentlichungsnummer: WO00048660

(56) Entgegenhaltungen:
- CH-A- 580 961
- US-A- 3 946 734
- US-A- 5 000 957
- US-A- 5 008 112
- US-A- 5 062 841

## Beschreibung

Die vorliegende Erfindung betrifft ein Implantat gemäß dem Oberbegriff des Anspruchs 1.

Hier ist unter dem Begriff "Implantat" zunächst im engeren Sinne ein in den Körper eines Tieres oder eines Menschen zumindest vorübergehend einzusetzendes Element zu verstehen, das beispielsweise ausschließlich therapeutische Funktionen aber auch Stütz- und/oder Gelenkfunktionen ausüben kann. Im weiteren Sinne sind hierunter jedoch auch mit dem Körper von außen, insbesondere vorübergehend in Kontakt bringbare Elemente o. dgl. zu verstehen.

Unter dem Begriff "therapeutisches Mittel" sind hier insbesondere Arzneimittel bzw. Pharmazeutika einerseits und Heilmittel und sonstige, dem menschlichen oder tierischen Körper zuzuführende Stoffe andererseits zu verstehen. Insbesondere kommen auch alle in der EP - A - 0 875 218 genannten, dort als "medication" bezeichneten therapeutischen Mittel bzw. Rezeptoragonisten, Rezeptorantagonisten, Enzyminhibitoren, Neurotransmitter, Zytostatika, Antibiotika, Hormone, Vitamine, Stoffwechselsubstrate, Antimetabolite, Diuretika und dergleichen als therapeutisches Mittel in Betracht.

Aus der DE - C - 197 04 497 ist eine implantierbare Infusionspumpe bekannt, bei der ein Arzneimittel mittels eines Treibmittels aus einem Aufnahmeraum ausgetrieben und über einen Katheter an den Körper abgegeben wird. Zwischen dem Aufnahme-raum und dem Katheter ist eine Drosselstrecke vorgesehen. Die Drosselstrecke ist durch eine Perfusionsplatte gebildet, die mit einer Vielzahl von Bohrungen in der Größenordnung von 1 µm versehen ist. Die Bohrungen sind mittels Laserstrahl in die beispielsweise aus Keramik bestehende Perfusionsplatte eingebracht.

Bei der bekannten Infusionspumpe strömt das Arzneimittel aufgrund des von dem Treibmittel verursachten Drucks durch die Bohrungen der Perfusionsplatte in den sich anschließenden Katheter. Die Perfusionsplatte wirkt hier als Drosselstelle, d. h. die pro Zeiteinheit durchströmende Menge an Arzneimittel hängt von dem Druck des Treibmittels und den fluidischen Eigenschaften des Arzneimittels ab. Bei dieser Durchströmung der Perfusionsplatte beschränkt sich die Wechselwirkung zwischen den Bohrungen der Perfusionsplatte und dem ausgetriebenen Arzneimittel auf die Drosselwirkung der Bohrungen, also auf einen strömungsmäßigen, quasi mechanischen Einfluß. Hierbei ist nachteilig, daß die pro Zeiteinheit abgegebene bzw. durchströmende Menge an Arzneimittel von dem durch das Treibmittel bewirkten Druck abhängt, so daß oftmals unvermeidliche Druckänderungen zu ungewollten Schwankungen der Abgabegeschwindigkeit führen. Weiterhin besteht der Nachteil, daß sich die Bohrungen der Perfusionsplatte bzw. sonstiger Drosseln durch Ablagerung von eingedrungenen Stoffen zumindest teilweise zusetzen können. Dies führt zu einer unerwünschten und undefinierten Änderung der Drosselwirkung und damit zu einer ungewünschten Beeinflussung der pro Zeiteinheit abgegebenen bzw. durchströmenden Menge an Arzneimittel.

Die US - A - 5,008,112 offenbart eine Vorrichtung zum kontinuierlichen Zuführen eines Mittels. Unter anderem ist hier ein mikroporöser Einsatz als Durchlaßelement vorgesehen, der insbesondere aus Kunststoff besteht. Durch die üblichen Verfahren sind nur verhältnismäßig grobe Poren im µm-Bereich herstellbar.

Die den Ausgangspunkt der vorliegenden Erfindung bildende US - A - 5,000,957 offenbart ein ähnliches Zuführsystem mit einem mikroporösen Wandungselement. Das mikroporöse Wandungselement weist eine Porengröße von 1 µm bis 100 µm auf und ist vorzugsweise aus Kunststoff hergestellt.

Der vorliegenden Erfindung liegt die Aufgabe zugrunde, ein Implantat bereitzustellen, das bei verhältnismäßig genau festlegbaren Eigenschaften des Durchlaßelements einfach bzw. kostengünstig herstellbar ist, wobei das Implantat eine sehr genaue, vorzugsweise druckunabhängige Abgabe eines Mittels ermöglicht.

Die obige Aufgabe wird durch ein Implantat gemäß Anspruch 1 gelöst. Vorteilhafte Weiterbildungen sind Gegenstand der Unteransprüche.

Ein Aspekt der vorliegenden Erfindung liegt darin, ein Diffusionselement mit offenen Poren vorzusehen, so daß nur eine Diffusion, jedoch keine freie Strömung ermöglicht wird, und/oder eine chemische Modifizierung von Porenwandungen vorzusehen, so daß eine vorzugsweise selektive Wechselwirkung hinsichtlich des Durchtritts mit einem therapeutischen Mittel bzw. mindestens einem Wirkstoff des therapeutischen Mittels erreicht wird. So kann eine zumindest weitgehende Unabhängigkeit der pro Zeiteinheit abgegebenen Menge von dem auf das therapeutische Mittel wirkenden Druck erreicht werden. Folglich wird eine genauere Dosierung möglich, insbesondere bei geringen Abgabemengen. Weiterhin wird ein Zusetzen bzw. Verstopfen des Durchlaßelements bei der erfindungsgemäßen Ausgestaltung zumindest weitestgehend ausgeschlossen.

Gemäß einer bevorzugten Ausgestaltung weisen die Poren des Durchlaßelements im Mittel einen Durchmesser von 20 nm bis 250 nm auf. Bei dieser Porengröße ist eine freie Durchströmung der Poren zumindest weitestgehend ausgeschlossen, so daß sich die gewünschte Druckunabhängigkeit der pro Zeiteinheit abgegebenen Menge einstellt. Zudem schließt diese Porengröße einen Eintritt von körpereigenen Stoffen, wie Proteinen, in die Poren und damit in das Implantat aus.

Zur chemischen Modifizierung können die Wandungen der Poren des Durchlaßelements beispielsweise hydrophil oder hydrophob ausgebildet und/oder zumindest bereichsweise mit funktionellen Gruppen versehen sein. So kann erreicht werden, daß beispielsweise nur das therapeutische Mittel oder nur ein Wirkstoff des therapeutischen Mittels durch die Poren hindurchtreten kann, so daß eine selektive Wechselwirkung zwischen den chemisch modifizierten Porenwandungen und dem therapeutischen Mittel bzw. mindestens einem Wirkstoff des therapeutischen Mittels erreichbar ist. Diese selektive Wechselwirkung kann ein ungewolltes Zusetzen bzw. Verstopfen der Poren verhindern.

Eine sehr einfache Herstellung und Ausbildung von hochgradig gleichförmigen Poren im Durchlaßelement wird durch eine künstliche, insbesondere elektrolytische, Oxidierung (Anodisierung), insbesondere von Aluminium, ermöglicht. Es eignen sich hierfür aber auch alle sogenannten Ventilmetalloxide, wie Aluminium-, Tantal-, Eisen-, Wolfram- und/oder Titanoxid, sowie Magnesiumoxid.

Durch Variation der elektrischen Spannung bei der Anodisierung können der Durchmesser der Poren und die Flächendichte der Poren, d. h. die Anzahl der Poren pro Fläche, variiert werden. Folglich kann die Form der Poren in weiten Bereichen gesteuert werden. Insbesondere sind die Poren zumindest im wesentlichen röhrenartig ausgebildet und erstrecken sich von der Oberfläche des Durchlaßelements im wesentlichen senkrecht durch das Durchlaßelement hindurch, wobei der Querschnitt der Poren und/oder deren Öffnung im Durchmesser bzw. in der Fläche abschnittsweise reduziert sein kann, um gewünschte Eigenschaften zu erhalten.

Eine ganz besonders bevorzugte Ausführungsform zeichnet sich durch eine zweite dem Aufnahmeraum zugeordnete Durchlaßöffnung auf, in die ebenfalls ein Durchlaßelement bzw. ein beispielsweise membranartiges Trennelement eingesetzt ist, so daß durch die eine Öffnung bzw. das darin eingesetzte Durchlaßelement das therapeutische Mittel bzw. mindestens ein Wirkstoff des therapeutischen Mittels hindurch aus dem Aufnahmeraum entweichen und Stoffe durch die andere Durchlaßöffnung bzw. das darin eingesetzte Durchlaß- bzw. Trennelement von außen in den Aufnahmeraum eindringen können. Diese quasi doppelte Osmose kann durch gezielte, unterschiedliche Ausbildung und/oder chemische Modifizierung der Durchlaßelemente erreicht werden. Die von außen in den Aufnahmeraum eindringenden Stoffe, wie Wasser o. dgl., können eine Volumenverringerung des therapeutischen Mittels im Aufnahmeraum ausgleichen, so daß kein den Austritt des therapeutischen Mittels aus dem Aufnahmeraum behindernder Unterdruck bzw. keine das Durchlaßelement zerstörende Druckdifferenz entsteht.

Bei Bedarf kann ein Wandelement zur Unterteilung des Aufnahmeraums vorgesehen sein, um eine Vermischung bzw. Verdünnung des therapeutischen Mittels durch in den Aufnahmeraum eindringende Stoffe zu vermeiden.

Nachfolgend wird die vorliegende Erfindung anhand der Zeichnung eines bevorzugten Ausführungsbeispiels näher erläutert. Es zeigt:
- Fig. 1: eine schematische Schnittdarstellung eines vorschlagsgemäßen Implantats;
- Fig. 2: eine schematische Schnittdarstellung einer Pore eines beidseitig abgestützten Durchlaßelements des Implantats gemäß Fig. 1; und
- Fig. 3, 4: elektronenmikroskopische Aufnahmen einer Aluminiumoxidschicht mit Poren in unterschiedlichen Vergrößerungen.

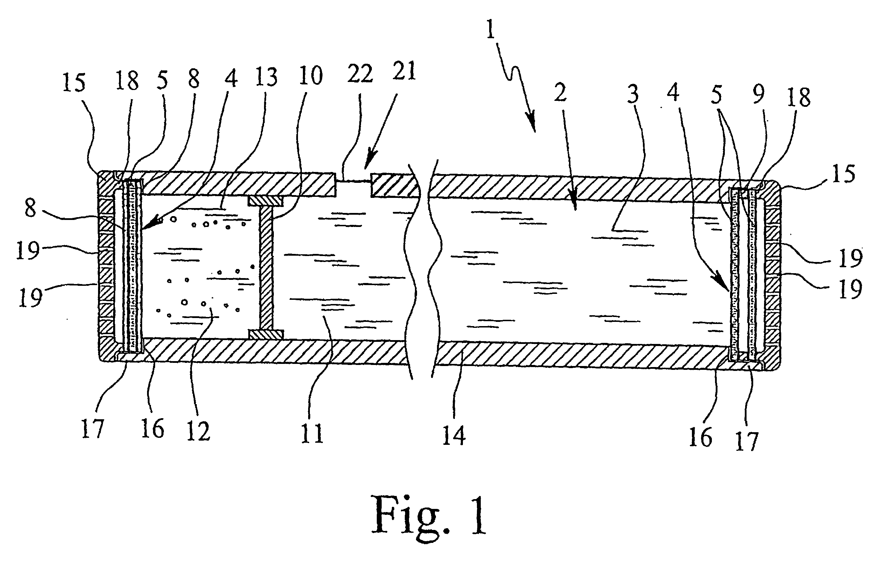

Fig. 1 zeigt in schematischer Schnittdarstellung ein vorschlagsgemäßes Implantat 1. Das Implantat 1 weist im dargestellten Ausführungsbeispiel eine im wesentlichen zylindrische Form auf. Jedoch sind auch beliebige andere Formen, wie flache oder scheibenförmige Formen, möglich.

Das Implantat 1 weist einen Aufnahmeraum 2 zur Aufnahme eines therapeutischen Mittels 3 auf. Hinsichtlich des therapeutischen Mittels 3 wird auf die eingangsseitige Definition verwiesen.

Das Implantat weist mindestens eine Durchlaßöffnung 4 auf, die hier insbesondere im Bereich eines Endes bzw. einer Stirnseite des Implantats 1 angeordnet ist. Der Durchlaßöffnung 4 ist mindestens ein Durchlaßelement 5 zugeordnet. Beim Darstellungsbeispiel sind zwei Durchlaßelemente 5 nacheinander in die rechte, mit dem therapeutischen Mittel 3 in Kontakt stehende Durchlaßöffnung 4 aus Sicherheitsgründen eingesetzt, um ein ungewolltes bzw. unkontrolliertes Entweichen des therapeutischen Mittels aus dem Aufnahmeraum 2 bei Bruch oder Beschädigung eines Durchlaßelements 5 mit Sicherheit auszuschließen. Die beiden Durchlaßelemente 5 sind beim Ausführungsbeispiel identisch ausgebildet, wobei jedoch auch eine unterschiedliche Ausbildung in Betracht kommt.

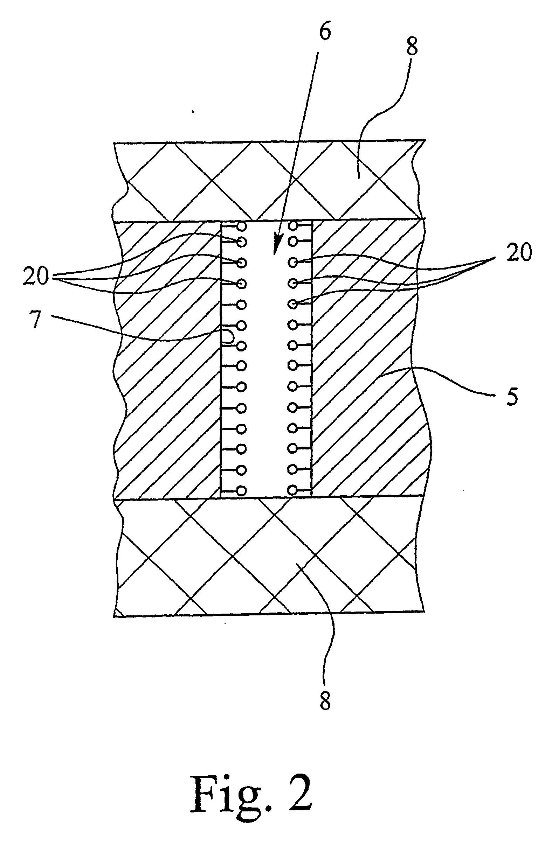

Nachfolgend wird die bevorzugte Ausbildung eines Durchlaßelements 5 anhand der schematischen Schnittdarstellung durch ein Durchlaßelement 5 gemäß Fig. 2 näher erläutert.

Das Durchlaßelement 5 ist für das therapeutische Mittel 3 bzw. mindestens einen Wirkstoff des therapeutischen Mittels 3 durchlässig. Hierzu ist das Durchlaßelement 5 vorzugsweise offenporig ausgebildet. Fig. 2 zeigt in der ausschnittsweisen Schnittdarstellung eine Pore 6. Das Durchlaßelement 5 weist eine Vielzahl derartiger Poren 6 auf, durch die das therapeutische Mittel 3 bzw. mindestens ein Wirkstoff des therapeutischen Mittels 3 aus dem Aufnahmeraum 2 hindurch nach außen treten, insbesondere nur diffundieren, kann.

Fig. 2 ist zu entnehmen, daß sich die Poren 6 im wesentlichen senkrecht zu der in Fig. 2 horizontal verlaufenden Haupterstreckungsebene des Durchlaßelements 5 durch dieses hindurch erstrecken. Die Poren 6 verlaufen dementsprechend im wesentlichen parallel zueinander. Insbesondere sind die Poren 6 im wesentlichen gleichförmig, insbesondere im wesentlichen kreiszylindrisch, ausgebildet.

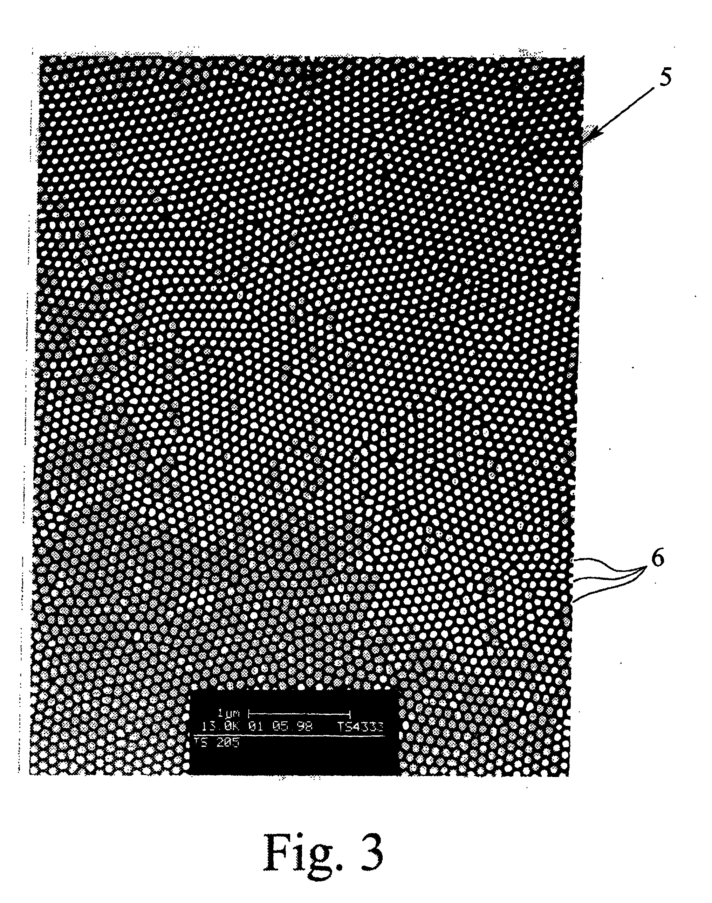

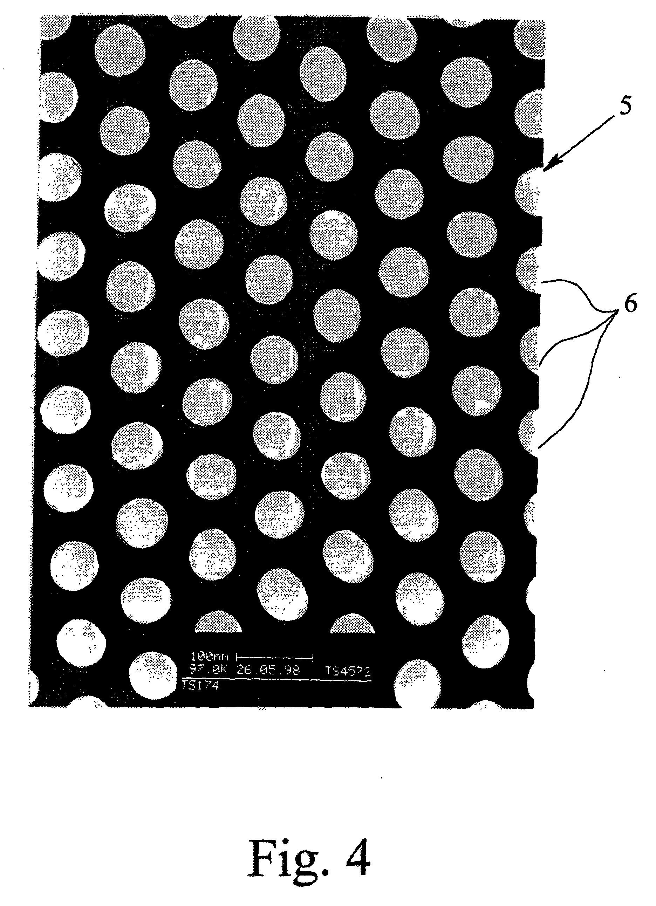

Fig. 3 und 4, die elektronenmikroskopische Aufnahmen einer Oberfläche eines Durchlaßelements 5 bei unterschiedlicher Vergrößerung darstellen, verdeutlichen, wie gleichmäßig verteilt und ausgebildet die hell erscheinenden, rohrförmigen Poren 6 sind.

Vorzugsweise beträgt die Flächendichte der Poren 6 etwa 10⁸ bis 10¹¹/cm². Der mittlere Porendurchmesser beträgt vorzugsweise maximal 500 nm, insbesondere 250 nm bis 20 nm.

Beim dargestellten Ausführungsbeispiel ist Fig. 2 zu entnehmen, daß die Poren 6 über ihre gesamte Erstreckung durch das Durchlaßelement 5 hindurch einen im wesentlichen konstanten Querschnitt aufweisen. Die Porenwandung 7 der Poren 6 bildet hier jeweils also im wesentlichen eine Zylindermantelfläche.

Das Durchlaßelement 5 weist eine geringe Dicke von insbesondere weniger als 50 µm, vorzugsweise maximal 5 µm, auf. Dementsprechend ergibt sich ein verhältnismäßig geringer Diffusions- bzw. Durchtrittswiderstand für das therapeutische Mittel 3 bzw. mindestens einen Wirkstoff des therapeutischen Mittels 3.

Das Durchlaßelement 5 besteht vorzugsweise zumindest im wesentlichen aus Aluminiumoxid, das insbesondere elektrolytisch abgeschieden bzw. gebildet wird. Beispielsweise wird eine Aluminiumschicht, die von einem nicht dargestellten Träger getragen ist, elektrisch oxidiert (anodisiert) und dann von dem Träger abgelöst, um das Durchlaßelement 5 zu erhalten. Bei der elektrolytischen Oxidierung kann der Durchmesser der Poren 6 sehr einfach durch entsprechende Einstellung der angelegten Spannung verändert werden. Hierbei ergibt sich etwa ein Durchmesser von 1,2 bis 1,4 nm pro 1 V anodischer Spannung.

Das Material des Durchlaßelements 5 bzw. nicht oxidiertes Material, wie Aluminium, kann alternativ beispielsweise durch Plasmabeschichten auf den nicht dargestellten Träger aufgebracht und ggf. anschließend oxidiert werden.
Die Herstellung des Durchlaßelements 5 ist jedoch nicht auf die voranstehenden Beispiele beschränkt, beispielsweise könnte auch eine Oxidierung einer entsprechenden Oberflächenschicht des nicht dargestellten Trägers in Betracht kommen, die dann abgelöst wird.

Des weiteren ist das Material für das Durchlaßelement nicht auf Aluminiumoxid beschränkt, sondern darüber hinaus sind generell alle sogenannten Ventilmetalloxide und Magnesiumoxid einsetzbar. Neben diesen Oxiden sind generell auch keramische Materialien geeignet, die im wesentlichen eine entsprechende Porenbildung aufweisen bzw. ermöglichen.

Aufgrund seiner geringen Dicke weist das Durchlaßelement 5 eine allenfalls geringe Eigenstabilität auf. Es ist daher vorzugsweise von mindestens einem, beispielsweise gitterartig ausgebildeten Halteelement 8 auf wenigstens einer Seite abgestützt. Fig. 2 zeigt eine Ausführungsalternative, bei der das Durchlaßelement 5 beidseitig von einem Halteelement 8 abgestützt ist, also zwischen zwei Halteelementen 8 gehalten ist.

Beim Darstellungsbeispiel gemäß Fig. 1 weist das Implantat 1 eine zweite Öffnung 4 auf, die vorzugsweise am anderen, hier linken Ende bzw. gegenüberliegend der ersten Öffnung 4 angeordnet ist. Dieser zweiten Durchlaßöffnung 4 ist vorzugsweise ebenfalls ein Durchlaßelement 5 gemäß der voranstehenden Beschreibung zugeordnet. Insbesondere ist dieses Durchlaßelement 5 auch in die Durchlaßöffnung 4 eingesetzt, so daß ein Stoffaustausch zwischen dem Aufnahmeraum 2 des Implantats 1 und dem das Implantat 1 umgebenden Außenraum ebenfalls nur durch das Durchlaßelement 5 hindurch möglich ist.

Beim Darstellungsbeispiel gemäß Fig. 1 ist der zweiten Durchlaßöffnung 4 lediglich ein einziges Durchlaßelement 5 zugeordnet, das entsprechend der Darstellung in Fig. 2 beidseitig von Halteelementen 8 abgestützt ist.

Auf der anderen Seite, bei der ersten Öffnung 4 sind demgegenüber als Ausführungsalternative die zwei Durchlaßelemente 5 durch einen vorzugsweise ringförmigen Abstandhalter 9 voneinander beabstandet gehalten. Zusätzlich können nicht dargestellte Halteelemente 8 oder sonstige Stützelemente den Durchlaßelementen 5 zugeordnet sein, um eine ausreichende Abstützung der Durchlaßelemente 5, insbesondere bei mangelnder Eigenstabilität und Belastbarkeit sicherzustellen.

Wie Fig. 1 zu entnehmen ist, weist das Implantat 1 ein hier im wesentlichen kolbenartig ausgebildetes Wandelement 10 auf, das den Aufnahmeraum 2 in einen ersten Raumabschnitt 11 und einen zweiten Raumabschnitt 12 unterteilt, wobei der erste Raumabschnitt 11 mit der ersten bzw. einer Durchlaßöffnung 4 in Verbindung steht und der zweite Raumabschnitt 12 mit der zweiten bzw. einer anderen Durchlaßöffnung 4 in Verbindung steht. Das Wandelement 10 ist hier kolbenartig verschieblich in den Aufnahmeraum 2 eingebaut. Jedoch kommt beispielsweise auch eine membranartige oder balgartige Ausbildung des Wandelements 10 bei entsprechender Flexibilität, Beweglichkeit und/oder Verschieblichkeit in Betracht.

Vorzugsweise ist das therapeutische Mittel 3 nur in dem ersten Raumabschnitt 11 eingefüllt. Im zweiten Raumabschnitt 12 ist vorzugsweise ein anderes Mittel, hier als Kompensationsmittel 13 bezeichnet, enthalten. Die Funktion des Kompensationsmittels 13 wird noch im einzelnen erläutert.

Ein einfaches Ein- bzw. Befüllen des Implantats 1 bzw. des Aufnahmeraums 2 mit dem therapeutischen Mittel 3 und dem optional vorgesehenen Kompensationsmittel 13 wird in bevorzugter Ausgestaltung dadurch ermöglicht, daß mindestens eine der Durchlaßöffnungen 4 zunächst noch offen ist oder geöffnet werden kann. Erst nach dem Füllen des Aufnahmeraums 2 wird dann das zugeordnete Durchlaßelement 5 in die Durchlaßöffnung 4 eingesetzt.

Insbesondere sind bei der vorgeschlagenen, aber nicht zwingenden zylindrischen Ausbildung des Implantats 1 die Durchlaßöffnungen 4 im Bereich der Enden, insbesondere über den gesamten Querschnitt, eines den Aufnahmeraum 2 bildenden, hohlzylindrischen Grundkörpers 14 ausgebildet. Weiter sind den Durchlaßöffnungen 4 insbesondere zum Schutz der eingesetzten Durchlaßelemente 5 vor äußeren mechanischen Einwirkungen Schutzabdekkungen 15 zugeordnet. Bei der zylindrischen Ausbildung des Implantats 1 und den stirnseitigen Durchlaßöffnungen 4 bietet sich dann eine endkappenseitige Ausbildung der Schutzabdeckungen 15 besonders an.

Nach dem Einsetzen der Durchlaßelemente 5 und Anbringen der Schutzabdeckungen 15 am hohlzylindrischen Körpers 14 des Implantats 1 sind die zugeordneten Durchlaßelemente 5 sowie eventuelle Halteelemente 8, Abstandhalter 9 und dergleichen in ihren gewünschten Lagen im Bereich der Durchlaßöffnung 4 fixiert. Insbesondere ist im Bereich jeder Durchlaßöffnung 4 eine an die Innenkontur der Durchlaßöffnung 4 angepaßte, hier ringförmige Schulter 16 gebildet, an die sich ein Abschnitt 17 mit vergrößertem Innendurchmesser vom Grundkörper 14 zur Aufnahme des mindestens einen Durchlaßelements 5 und zugeordneter Halteelemente 8, Abstandhalter 9 und dergleichen anschließt. Die zugeordnete Schutzabdeckung 15 weist einen zylindrischen Ansatz 18 auf, der derart an den Abschnitt 17 mit vergrößertem Innendurchmesser angepaßt ist, daß der Ansatz 18 im Preßsitz in den Abschnitt 17 einsteckbar ist, so daß die Schutzabdekkung 15 vorzugsweise ohne weitere Sicherungsmittel durch den Preßsitz am Grundkörper 14 quasi unlösbar angebracht ist, wobei hier der Ansatz 18 das Durchlaßelement 5 bzw. die Durchlaßelemente 5 und eventuelle Halteelemente 8 und Abstandhalter 9 und dergleichen der zugeordneten Durchlaßöffnung 4 zwischen sich und der zugeordneten Schulter 16 hält und damit im Abschnitt 17 fixiert.

Es ist selbstverständlich, daß jede Durchlaßöffnung 4 auch eine von der Kreisform abweichende Umfangskontur aufweisen kann. Das zugeordnete bzw. darin eingesetzte Durchlaßelement 5 weist dann eine dementsprechend bzw. an den jeweiligen Abschnitt 17 angepaßte Außenkontur auf.

Die Schutzabdeckung 15 weist Durchgangsöffnungen 19 auf, die im Vergleich zu den Poren 6 einen großen Durchmesser aufweisen, so daß eine zumindest im wesentlichen ungestörte Strömung durch die Schutzabdeckung 15 hindurch möglich ist. Die Schutzabdeckungen 15 dienen nämlich neben der hier vorgesehenen Fixierung der Durchlaßelemente 5 und zugeordneter Bauteile primär einem Schutz der zugeordneten Durchlaßelemente 5 vor mechanischen Einwirkungen, die zu einer Beschädigung oder Zerstörung der relativ spröden Durchlaßelemente 5 führen könnten.

Der Grundkörper 14 und die Schutzabdeckungen 15 sind vorzugsweise aus einem körpergeeigneten Material, vorzugsweise Metall, hergestellt.

Nach dem Befüllen des Implantats 1 mit dem therapeutischen Mittel 3 und dem Kompensationsmittel 13 und nach dem Verschließen der Durchlaßöffnungen 4 durch die Durchlaßelemente 5 und deren Fixierung und Abdekkung durch die Schutzabdekkungen 15 wird das Implantat 1 implantiert. Das therapeutische Mittel 3 bzw. mindestens ein Wirkstoff des therapeutischen Mittels 3 kann dann durch das mindestens eine Durchlaßelement 5, hier durch die beiden Durchlaßelemente 5 der mit dem ersten Raumabschnitt 11 in Verbindung stehenden, ersten Durchlaßöffnung 4 hindurch diffundieren und in den das Implantat 1 umgebenden, nicht dargestellten Körper durch die Durchgangsöffnungen 19 hindurch austreten. Die beiden Durchlaßelemente 5 der ersten Durchlaßöffnung 4 weisen hierzu Poren 6 auf, deren Porengröße und/oder deren Porenwandung 7 derart ausgebildet ist bzw. sind, daß zumindest im wesentlichen lediglich eine Diffusion des therapeutischen Mittels 3 oder des gewünschten Wirkstoffs des therapeutischen Mittels 3 durch die Durchlaßelemente 5 hindurch aus dem ersten Raumabschnitt 11 des Aufnahmeraums 2 heraus auftritt.

Um die vorgenannte, vorzugsweise selektive Diffusion zu erreichen, ist die Größe der Poren 6 entsprechend angepaßt und/oder ist die Porenwandung 7 mittels in Fig. 2 angedeuteter Wechselwirkungspartner 20 entsprechend chemisch modifiziert. Die Wechselwirkungspartner 20 sind vorzugsweise auf der Porenwandung 7 zumindest bereichsweise fixiert und bewirken beispielsweise eine hydrophobe oder hydrophile Eigenschaft der Poren 6 oder wirken als funktionelle Gruppen, um vorzugsweise nur einen selektiven Durchtritt durch die Durchlaßelemente 5 zu ermöglichen, also im wesentlichen die Wirkung einer semipermeablen Membran zu erreichen.

Als funktionelle Gruppen kommen beispielsweise Amin-, Mercapto-, Carboxy-, Hydroxygruppen und/oder organisch modifizierte Silane in Betracht.

Um die Verringerung des Volumens des therapeutischen Mittels 3 bei fortschreitender Abgabe des therapeutischen Mittels 3 bzw. mindestens eines Wirkstoffs des therapeutischen Mittels 3 zu kompensieren, ist das Durchlaßelement 5 der zweiten Durchlaßöffnung 4, die mit dem zweiten Raumabschnitt 12 des Aufnahmeraums 2 in Verbindung steht, derart ausgebildet, daß wenigstens ein Stoff, beispielsweise Wasser, aus dem nicht dargestellten, das Implantat 1 umgebenden Körper durch das Durchlaßelement 5 in den zweiten Raumabschnitt 12 eindringen und sich ggf. mit dem optional vorgesehenen Kompensationsmittel 13 vermischen kann. Je nach Ausbildung des Durchlaßelementes 5 der zweiten Durchlaßöffnung 4 kann der genannte Eindringprozeß auch ohne das Kompensationsmittel 13 ablaufen. In jedem Fall verhindert das hier verschieblich ausgebildete Wandelement 10 dabei eine ungewollte Verdünnung des therapeutischen Mittels 13 und wird entsprechend der Volumenveränderung in den Raumabschnitten 11 und 12 verschoben.

Bei dem Kompensationsmittel 13 kann es sich beispielsweise um eine Kochsalzlösung handeln.

Aus dem Vorgenannten ergibt sich, daß bei dem dargestellten Ausführungsbeispiel quasi eine doppelte Osmose erfolgt, einerseits tritt das therapeutische Mittel 3 bzw. mindestens ein Wirkstoff des therapeutischen Mittels 3 aus dem Aufnahmeraum 2 aus und andererseits tritt ein geeigneter Stoff in den Aufnahmeraum 2 durch die zweite Durchlaßöffnung 4 bzw. das dieser zugeordnete Durchlaßelement 5 in den Aufnahmeraum 2 ein.

Aus dem Vorgenannten ergibt sich weiter, daß zumindest im wesentlichen lediglich eine Diffusion eines geeigneten Stoffes aus dem nicht dargestellten, das Implantat 1 umgebenden Körper in den zweiten Raumabschnitt 12 vorgesehen ist. Insbesondere ist daher das Durchlaßelement 5 auf dieser Eintrittsseite (linke Seite in Fig. 1) gegenüber dem mindestens einen Durchlaßelement 5 auf der Austrittsseite (rechte Seite in Fig. 1) unterschiedlich - insbesondere hinsichtlich Porengröße, Porendichte sowie chemischer Modifizierung der Porenwandungen 7 - ausgebildet. Nachfolgend werden diesbezügliche Ausführungsbeispiele näher erläutert.

Durch die Verwendung von z. B. organisch modifizierten Silanen kann die Polarität der Poren 6 in idealer Weise variiert werden. Weiter kann die Austrittsgeschwindigkeit der vom Implantat 1 abzugebenden Substanz - therapeutisches Mittel 3 oder mindestens ein Wirkstoff des therapeutischen Mittels 3 - durch die Porengröße, Porendichte sowie die chemische Modifizierung der Porenwandungen 7 gesteuert werden.

Wenn eine hydrophobe Substanz mit hoher Dosis, wie Steroide, trizyklische Antidepressiva o. dgl., vom Implantat 1 abgegeben werden soll, sind auf der Austrittsseite große Poren 6 mit hydrophober Innenbeschichtung und auf der Eintrittsseite kleine Poren 6 mit hydrophiler Innenbeschichtung zur Aufnahme von Wasser vorgesehen.

Wenn eine hydrophobe Substanz mit geringer Dosis vom Implantat 1 abgegeben werden soll, sind dementsprechend kleinere Poren 6 vorgesehen.

Wenn eine hydrophile Substanz mit hoher Dosis vom Implantat 1 abgegeben werden soll, sind vorzugsweise große, hydrophile Poren 6 auf der Austrittsseite und kleine, hydrophile Poren 6 zur Aufnahme von Wasser auf der Eintrittsseite vorgesehen.

Anstelle des vorzugsweise vorgesehenen, offenporigen Durchlaßelements 5 kann dem zweiten Raumabschnitt 12 bzw. der zweiten Durchlaßöffnung 4 auch ein nicht offenporiges Trennelement, wie eine porenfreie, beispielsweise semipermeable Membran, zugeordnet sein, durch das ein Stoffaustausch stattfinden kann.

Gegebenenfalls kann das Wandelement 10 auch vollständig entfallen, wenn eine Verdünnung des therapeutischen Mittels 3 unkritisch ist, beispielsweise wenn die Diffusion eines gewünschten Wirkstoffs durch das Durchlaßelement 5 aus dem Aufnahmeraum 2 nach außen zumindest nicht wesentlich durch eine Verdünnung beeinflußt wird. In diesem Fall ist der Aufnahmeraum 2 also nicht unterteilt. Das Kompensationsmittel 13 kann dann entsprechend entfallen.

Andererseits kann bei ausreichender Abdichtwirkung des Wandelements 10, beispielsweise in Form einer flexiblen Membran oder eines Balgs, das Implantat 1 im Bereich der zweiten Durchlaßöffnung 4 eine freie Strömung in und aus dem zweiten Raumbereich 12 durch die Durchgangsöffnungen 19 hindurch gestatten, das in die zweite Durchlaßöffnung 4 eingesetzte Durchlaßelement 5 also entfallen, so daß sich das Volumen des ersten Raumabschnitts 11 frei und bedarfsgerecht an das Volumen des therapeutischen Mittels 3 durch entsprechende Verschiebung und/oder Verformung des Wandelements 10 anpassen kann.

Gemäß einer weiteren Ausführungsalternative kann das Wandelement 10 insbesondere bei flexibler Ausbildung eine Außenwandung des Implantats 1 bzw. des Aufnahmeraums 2 bilden. In diesem Fall können der zweite Raumabschnitt 12, die zweite Durchlaßöffnung 4 mit zugeordnetem Durchlaßelement 5 und die zugeordnete Schutzabdeckung 15 vollständig entfallen.

Gegebenenfalls kann auch bei zumindest im wesentlichen starrer Ausbildung des Aufnahmeraums 2, also bei im wesentlichen unveränderlichem Volumen des Aufnahmeraums 2, lediglich eine Durchlaßöffnung 4 mit mindestens einem zugeordneten Durchlaßelement 5 genügen. In diesem Fall kann einerseits das therapeutische Mittel 3 bzw. mindestens ein Wirkstoff des therapeutischen Mittels 3 durch das Durchlaßelement 5 hindurch aus dem Aufnahmeraum 2 herausdiffundieren und andererseits ein Stoff, beispielsweise Wasser, aus dem das Implantat 1 umgebenden Körper durch das Durchlaßelement 5 hindurch in den Aufnahmeraum 2 hineindiffundieren. Um diesen Ein- und Austritt durch das gleiche Durchlaßelement 5 zu ermöglichen, sind vorzugsweise eine bestimmte Anzahl von Poren 6 unterschiedlich ausgebildet und/oder unterschiedlich chemisch modifiziert im Vergleich zu den anderen Poren 6.

Alternativ kann aber ein Eintritt und Austritt auch bei nur gleichartig ausgebildeten und/oder chemisch modifizierten Poren 6 erfolgen.

Außerdem können einer Durchlaßöffnung 4 auch zwei nebeneinander angeordnete, also parallel geschaltete Durchlaßelemente 5 unterschiedlicher Ausbildung zugeordnet sein.

Bei unveränderlichem Volumen des Aufnahmeraums 2 ist es wesentlich, daß die Druckbelastung auf das verhältnismäßig spröde Durchlaßelement 5 minimal gehalten wird. Dementsprechend ist ein entsprechendes Gleichgewicht der Volumenströme in Austrittsrichtung und Eintrittsrichtung vorzusehen. Dies gilt sowohl bei lediglich einer Durchlaßöffnung 4 als auch bei mehreren Durchlaßöffnungen 4 mit optionaler Unterteilung durch ein Wandelement 10, wie in Fig. 1 dargestellt.

Bei Bedarf kann das Implantat 1 auch ein Septum 21, wie in Fig. 1 angedeutet, aufweisen. Das Septum 21 kann einem anfänglichen Einfüllen und/oder Nachfüllen des therapeutischen Mittels 3 oder des Kompensationsmittels 13 dienen. Gegebenenfalls können auch zwei oder mehr Septa 21 vorgesehen sein.

Bei dem Septum 21 handelt es sich um ein aus dem Stand der Technik bereits bekanntes Element, das eine Membran 22 aufweist, die von einer entsprechend angepaßten Kanüle zum Ein- bzw. Nachfüllen des Aufnahmeraums 2 durchstochen werden kann und sich anschließend wieder selbsttätig dicht verschließt.

Bedarfsweise können die Poren 6 insbesondere auf der Außenseite des Durchlaßelements 5 temporär, beispielsweise durch eine manuell lösbare oder sich im implantierten Zustand selbstätig lösende Abdeckung, insbesondere bei längerer Lagerung des Implantats 1, zu Schutzzwecken überdeckt sein. Hierfür eignet sich beispielsweise eine sterile Folie.

## Patentansprüche

1. Implantat (1) mit einem Aufnahmeraum (2) für ein therapeutisches und/oder mindestens einen Wirkstoff enthaltendes Mittel (3), wobei der Aufnahmeraum (2) mindestens eine Durchlaßöffnung (4) aufweist, in die ein Durchlaßelement (5) eingesetzt ist, durch welches das Mittel (3) den Aufnahmeraum (2) verlassen kann,
wobei das Durchlaßelement (5) als ein Diffusionselement mit offenen Poren (6) mit einer Porengröße und/oder Porenwandung (7) ausgebildet ist, die zumindest im wesentlichen nur eine Diffusion des Mittels (3) und /oder mindestens eines Wirkstoffs des Mittels (3) durch das Diffusionselement gestattet, ohne eine freie Strömung durch das Durchlaßelement (5) zu ermöglichen, und/oder
wobei das Durchlaßelement (5) offene Poren (6) mit Porenwandungen (7) aufweist, die zumindest bereichsweise chemisch modifiziert sind, um mit dem Mittel (3), mit mindestens einem Wirkstoff des Mittels (3) und/oder mit außen befindlichen Stoffen, vorzugsweise selektiv, hinsichtlich des Durchtritts durch das Durchlaßelement (5) zuwechselwirken,
**dadurch gekennzeichnet,**
**daß** das Durchlaßelement (5) zumindest im wesentlichen aus durch Anodisieren hergestelltem Aluminium-, Magnesium-, Tantal-, Eisen-, Wolfram- und/oder Titanoxid besteht.

2. Implantat nach Anspruch 1, **dadurch gekennzeichnet, daß** das Durchlaßelement (5) membranartig oder folienartig ausgebildet ist und/oder im wesentlichen gleichmäßig dick ausgebildet ist und/oder eine Dicke von maximal 50 µm, insbesondere maximal 5 µm, aufweist.

3. Implantat nach einem der voranstehenden Ansprüche, **dadurch gekennzeichnet, daß** das Durchlaßelement (5) von mindestens einem vorzugsweise gitterartigen Halteelement (8) flächig abgestützt, insbesondere zwischen zweien gehalten ist und/oder daß in die Durchlaßöffnung (4) zwei Durchlaßelemente (5) nacheinander eingesetzt sind.

4. Implantat nach einem der voranstehenden Ansprüche, **dadurch gekennzeichnet, daß** die Poren (6) im wesentlichen gleichmäßig und/oder mit einer Flächendichte von 10⁸ bis 10¹¹/cm² über das Durchlaßelement (5) verteilt sind und/oder daß die Poren (6) im wesentlichen zylindrisch und/oder zueinander beabstandet und/oder gleichförmig ausgebildet sind und/oder im wesentlichen senkrecht zur Erstreckungsebene durch das Durchlaßelement (5) verlaufen und/oder jeweils Abschnitte mit unterschiedlichen bzw. zu- und/oder abnehmenden Querschnitten aufweisen.

5. Implantat nach einem der voranstehenden Ansprüche, **dadurch gekennzeichnet, daß** der Porendurchmesser im Mittel weniger als 500 nm, vorzugsweise weniger als 250 nm, insbesondere 250 bis 20 nm, beträgt und/oder daß die Poren (6) insbesondere außenseitig temporär überdeckt bzw. verschlossen sind.

6. Implantat nach einem der voranstehenden Ansprüche, **dadurch gekennzeichnet, daß** zur chemischen Modifizierung die Porenwandungen (7) zumindest bereichsweise hydrophil oder hydrophob ausgebildet und/oder zumindest bereichsweise mit funktionellen Gruppen, wie Amin-, Mercapto-, Carboxy- und/oder Hydroxygruppen, und/oder organisch modifizierten Silanen, versehen sind.

7. Implantat nach einem der voranstehenden Ansprüche, **dadurch gekennzeichnet, daß** das Implantat (1) ein flexibles und/oder bewegliches Wandelement (10) aufweist, das den Aufnahmeraum (2) abgrenzt, so daß das Volumen des Aufnahmeraums (2) veränderlich, insbesondere je nach Verringerung des Volumens des darin befindlichen Mittels (3) verringerbar ist, und/oder den Aufnahmeraum (2) unterteilt in einen ersten, mit einer ersten Durchlaßöffnung (4) in Verbindung stehenden Raumabschnitt (11) für das Mittel (3) und in einen zweiten, mit einer zweiten Durchlaßöffnung (4) in Verbindung stehenden Raumabschnitt (12) für ein Kompensationsmittel (13), so daß das Volumen des ersten Raumabschnitts (11) veränderlich, insbesondere je nach Verringerung des Volumens des darin befindlichen, therapeutischen Mittels (3) verringerbar ist, insbesondere wobei das Wandelement (10) membranartig, balgartig und/oder kolbenartig, insbesondere im Aufnahmeraum (2) verschieblich, ausgebildet ist.

8. Implantat nach einem der voranstehenden Ansprüche, **dadurch gekennzeichnet, daß** das Implantat (1) zwei separate, den Aufnahmeraum (2) nach außen öffnende Durchlaßöffnungen (4) aufweist, wobei in eine Durchlaßöffnung (4) ein erstes Durchlaßelement (5) und in die andere Durchlaßöffnung (4) ein vorzugsweise porenfreies Trennelement, wie eine semipermeable Membran, oder ein zweites Durchlaßelement (5) eingesetzt sind, wobei das erste Durchlaßelement (5) und das Trennelement bzw. das zweite Durchlaßelement (5) so ausgebildet sind, daß sie mit dem Mittel (3), mit mindestens einem Wirkstoff des Mittels (3) und/oder mit außen befindlichen Stoffen, vorzugsweise selektiv, hinsichtlich des Durchtritts unterschiedlich wechselwirken, insbesondere derart, daß das Mittel (3) und/oder mindestens ein Wirkstoff des Mittels (3) durch das erste Durchlaßelement (5) aus dem Aufnahmeraum (2) entweichen und daß mindestens ein Stoff, beispielsweise Wasser, durch das Trennelement bzw. das zweite Durchlaßelement (5) in den Aufnahmeraum (2) eindringen kann.

9. Implantat nach einem der voranstehenden Ansprüche, **dadurch gekennzeichnet, daß** das Implantat (1) einen länglichen, insbesondere im wesentlichen zylindrischen Grundkörper (14), eine dem Durchlaßelement (5) zugeordnete Schutzabdeckung (15) und/oder mindestens ein Septum (21), das mit dem Aufnahmeraum (2) und/oder mindestens einem Raumabschnitt (11, 12) des Aufnahmeraums (2) in Verbindung steht, aufweist.

10. Implantat nach einem der voranstehenden Ansprüche, **dadurch gekennzeichnet, daß** mindestens eine Durchlaßöffnung (4) an einem Stirnende eines Grundkörpers (14) ausgebildet ist, wobei vorzugsweise eine Schutzabdeckung (15) am Grundkörper (14) über einem in die Durchlaßöffnung (4) eingesetzten Durchlaßelement (5) oder Trennelement, insbesondere mittels Preßsitz und/oder lösbar, angebracht ist.

## Claims

1. Implant (1) with a receptacle space (2) for a therapeutic agent (3) and/or an agent (3) containing at least one active substance, wherein the receptacle space (2) comprises at least one passage opening (4), in which a permeable element (5) is inserted, through which the agent (3) can leave the receptacle space (2),
wherein the permeable element (5) is formed as a diffusion element with open pores (6) having a pore size and/or pore wall (7) which allows, at least essentially, only a diffusion of the agent (3) and/or of at least one active substance of the agent (3) through the diffusion element, without allowing any free flow through the permeable element (5), and/or
wherein the permeable element (5) comprises open pores (6) with pore walls (7) which are chemically modified at least regionally in order to interact with the agent (3), with at least one active substance of the agent (3) and/or with externally present substances, preferably selectively, in regard to the passage through the permeable element (5),
**characterized in**
**that** the permeable element (5) consists at least essentially of aluminum oxide, magnesium oxide, tantalum oxide, iron oxide, tungsten oxide, and/or titanium oxide, produced by anodization.

2. Implant according to claim 1, **characterized in that** the permeable element (5) is formed membrane-like or film-like and/or is formed essentially uniformly thick and/or has a thickness of a maximum of 50 µm, preferably a maximum of 5 µm.

3. Implant according to any one of the preceding claims, **characterized in that** the permeable element (5) is supported two-dimensionally by at least one, preferably grate-like, support element (8), preferably held between two of them, and/or that two permeable elements (5) are inserted one after another in the passage opening (4).

4. Implant according to any one of the preceding claims, **characterized in that** the pores (6) are distributed essentially uniformly and/or with a surface density of 10⁸ to 10¹¹/cm² over the permeable element (5), and/or that the pores (6) are essentially cylindrical and/or spaced at intervals from one another and/or uniformly formed and/or run through the permeable element (5) essentially perpendicularly to the plane of extension and/or respectively comprise portions with differing or widening and/or narrowing cross-sections.

5. Implant according to any one of the preceding claims, **characterized in that** the pore diameter is, on average, less than 500 nm, preferably less than 250 nm, particularly 20 nm to 250 nm, and/or that the pores (6) are temporarily covered or closed, particularly externally.

6. Implant according to any one of the preceding claims, **characterized in that**, for chemical modification, the pore walls (7) are formed, at least regionally, as hydrophilic or hydrophobic, and/or are, at least regionally, provided with functional groups, such as amine groups, mercapto groups, carboxy groups, and/or hydroxy groups, and/or organically modified silanes.

7. Implant according to any one of the preceding claims, **characterized in that** the implant (1) has a flexible and/or movable wall element (10), which delimits the receptacle space (2), so that the volume of the receptacle space (2) is variable, particularly reducible depending on the reduction of the volume of the agent (3) therein, and/or divides the receptacle space (2) into a first space portion (11) for the agent (3), connected with a first passage opening (4), and into a second space portion (12) for a compensation agent (13), connected with a second passage opening (4), so that the volume of the first space portion (11) is variable, particularly reducible depending on the reduction of the volume of the therapeutic agent (3) therein, particularly wherein the wall element (10) is formed like a membrane, a bellows, and/or a piston, particularly so that it is displaceable in the receptacle space (2).

8. Implant according to any one of the preceding claims, **characterized in that** the implant (1) comprises two separate passage openings (4) which open the receptacle space (2) to the outside, wherein a first permeable element (5) is inserted in one passage opening (4), and a preferably pore-free separating element, such as a semi-permeable membrane, or a second permeable element (5) is inserted in the other passage opening (4), wherein the first permeable element (5) and the separating element/the second permeable element (5) are formed in such a way that that they interact with the agent (3), with at least one active substance of the agent (3), and/or with externally located substances differently, preferably selectively, in regard to the passage, particularly such that the agent (3) or at least one active substance of the agent (3) can leave the receptacle space (2) through the first permeable element (5) and at least one substance, for example water, can penetrate into the receptacle space (2) through the separating element/the second permeable element (5).

9. Implant according to any one of the preceding claims, **characterized in that** the implant (1) comprises an oblong, particularly essentially cylindrical main body (14), a protective covering (15) associated to the permeable element (5), and/or at least one septum (21), which is connected with the receptacle space (2) and/or at least one space portion (11, 12) of the receptacle space (2).

10. Implant according to any one of the preceding claims, **characterized in that** at least one passage opening (4) is formed on one face of a main body (14), wherein preferably a protective covering (15) is provided at the main body (14) over a permeable element (5) or separating element inserted in the passage opening (4), particularly by means of a press fit or removably.

## Revendications

1. Implant (1) comportant une chambre de réception (2) pour un agent thérapeutique (3) et/ou contenant au moins une substance active, dans lequel la chambre de réception (2) présente au moins une ouverture de transport (4) dans laquelle est inséré un élément de transport (5) à travers lequel l'agent (3) peut quitter la chambre de réception (2),
dans lequel l'élément de transport (5) est réalisé sous la forme d'un élément de diffusion comportant des pores ouverts (6) à l'aide d'une porosité et/ou d'une paroi poreuse (7) qui permet, au moins à titre essentiel, uniquement une diffusion de l'agent (3) et/ou d'une substance active de l'agent (3) à travers l'élément de diffusion, sans autoriser un écoulement libre à travers l'élément de transport (5), et/ou
dans lequel l'élément de transport (5) représente des pores (6) comportant des parois poreuses (7) qui ont été modifiées par voie chimique, au moins dans certaines zones, de façon à obtenir une interaction avec l'agent (3), avec au moins une substance active de l'agent (3) et/ou avec des substances se trouvant à l'extérieur, de préférence sélective en ce qui concerne le passage à travers l'élément de transport (5),
**caractérisé en ce que** l'élément de transport (5) est constitué, au moins de manière essentielle, par de l'oxyde d'aluminium, de l'oxyde de magnésium, de l'oxyde de tantale, de l'oxyde de fer, de l'oxyde de tungstène et/ou de l'oxyde de titane préparé par anodisation.

2. Implant selon la revendication 1, **caractérisé en ce que** l'élément de transport (5) est réalisé sous la forme d'une membrane ou sous la forme d'une feuille et/ou est réalisé de telle sorte qu'il présente une épaisseur essentiellement uniforme et/ou présente une épaisseur maximale de 50 µm, en particulier une épaisseur maximale de 5 µm.

3. Implant selon l'une quelconque des revendications précédentes, **caractérisé en ce que** l'élément de transport (5) est supporté sur toute sa surface par au moins un élément de maintien (1) de préférence en forme de grille, en particulier est maintenu entre deux éléments de ce type et/ou **en ce qu'**on met en oeuvre, de manière successive, deux éléments de transport (5) dans l'ouverture de transport (4).

4. Implant selon l'une quelconque des revendications précédentes, **caractérisé en ce que** les pores (6) sont distribués de manière essentiellement uniforme et/ou avec une densité en surface de 10⁸ à 10¹¹/cm² par-dessus l'élément de transport (5) et/ou **en ce que** les pores (6) sont réalisés en une forme essentiellement cylindrique et/ou à l'écart les uns des autres et/ou sous une forme identique et/ou s'étendent essentiellement perpendiculairement au plan de projection à travers l'élément de transport (5) et/ou représentent respectivement des tronçons possédant des sections transversales différentes, respectivement qui augmentent et/ou qui diminuent.

5. Implant selon l'une quelconque des revendications précédentes, **caractérisé en ce que** le diamètre des pores est inférieur en moyenne à 500 nm, de préférence inférieur à 250 nm, en particulier s'élève de 250 à 20 nm, et/ou **en ce que** les pores (6) sont temporairement recouverts, respectivement obturés, en particulier du côté externe.

6. Implant selon l'une quelconque des revendications précédentes, **caractérisé en ce que**, pour la modification chimique, les parois des pores (7) sont réalisées, au moins dans certaines zones, pour présenter un caractère hydrophile ou hydrophobe et/ou sont munies, au moins dans certaines zones, de groupes fonctionnels tels que des groupes amines, des groupes mercapto, des groupes carboxyle et/ou des groupes hydroxyle, et/ou de silanes soumis à une modification organique.

7. Implant selon l'une quelconque des revendications précédentes, **caractérisé en ce que** l'implant (1) présente un élément de paroi flexible et/ou mobile (10) qui délimite la chambre de réception (2) de telle sorte que le volume de la chambre de réception (2) peut être modifié, en particulier peut être soumis à une réduction en fonction respectivement de la réduction du volume de l'agent (3) qui s'y trouve, et/ou subdivise la chambre de réception (2) en une première section de chambre (11) destinée à l'agent (3) et mise en liaison avec une première ouverture de transport (4) et en une deuxième section de chambre (12) destinée à un agent de compensation (13) qui est mise en liaison avec une deuxième ouverture de transport 4, de telle sorte que le volume de la première section de chambre (11) peut être modifié, en particulier peut être soumis à une réduction en fonction respectivement de la réduction du volume de l'agent (3) qui s'y trouve, en particulier dans lequel l'élément de paroi (10) est réalisé sous la forme d'une membrane, sous la forme d'un soufflet et/ou sous la forme d'un piston, en particulier est réalisé pour pouvoir se déplacer dans la chambre de réception (2).

8. Implant selon l'une quelconque des revendications précédentes, **caractérisé en ce que** l'implant (1) présente deux ouvertures de transport séparées (4) ouvrant la chambre de réception (2) vers l'extérieur, dans lequel, on met en oeuvre, dans une ouverture de transport 4, un premier élément de transport (5) et, dans l'autre ouverture de transport (4), un élément de séparation de préférence non poreux, tel que par exemple une membrane semi-perméable, ou un deuxième élément de transport (5), dans lequel le premier élément de transport (5) et l'élément de séparation, respectivement le deuxième élément de transport (5) sont réalisés de telle sorte qu'ils entrent en interaction de manière différente en ce qui concerne le passage, de préférence de manière sélective, avec l'agent (3), avec au moins une substance active de l'agent (3) et/ou avec des substances se trouvant à l'extérieur, en particulier de telle sorte que l'agent (3) et/ou au moins une substance active de l'agent (3) s'échappe à travers le premier élément de transport (5) hors de la chambre de réception (2) et de telle sorte qu'au moins une substance, par exemple de l'eau, est en mesure de pénétrer, à travers l'élément de séparation, respectivement à travers l'élément de transport (5), dans la chambre de réception (2).

9. Implant selon l'une quelconque des revendications précédentes, **caractérisé en ce que** l'implant (1) présente un corps de base oblong (14), en particulier essentiellement cylindrique, un recouvrement de protection (15) attribué à l'élément de transport (5) et/ou au moins une cloison (21) qui est mise en liaison avec la chambre de réception (2) et/ou avec au moins une section de chambre (11, 12) de la chambre de réception (2).

10. Implant selon l'une quelconque des revendications précédentes, **caractérisé en ce qu'**au moins une ouverture de transport (4) est réalisée, en particulier en ajustage serré et/ou de manière amovible, à l'extrémité frontale du corps de base (14), de préférence un recouvrement de protection (15) étant appliqué sur le corps de base (14) par-dessus un élément de transport (5) ou un élément de séparation inséré dans l'ouverture de transport (4).
